(19) Europäisches Patentamt
European Patent Office
Office européen des brevets

(11) Veröffentlichungsnummer: **0 011 272**
**B1**

(12) **EUROPÄISCHE PATENTSCHRIFT**

(45) Veröffentlichungstag der Patentschrift: **16.02.83**

(21) Anmeldenummer: **79104452.2**

(22) Anmeldetag: **12.11.79**

(51) Int. Cl.³: **C 07 C 45/50,**
C 07 C 47/225,
C 07 C 47/115,
C 07 C 47/42, C 11 B 9/00

# (54) Verfahren zur Herstellung von Aldehyden und Verwendung der Verfahrensprodukte als Riechstoffe.

(30) Priorität: **16.11.78 DE 2849742**

(43) Veröffentlichungstag der Anmeldung:
**28.05.80 Patentblatt 80/11**

(45) Bekanntmachung des Hinweises auf die Patenterteilung:
**16.02.83 Patentblatt 83/7**

(84) Benannte Vertragsstaaten:
**BE CH DE FR GB IT SE**

(56) Entgegenhaltungen:
**EP - A - 0 008 459**
**EP - A - 0 011 273**
**DE - A - 1 939 322**
**DE - C - 956 754**
**FR - A - 2 224 428**
**FR - A - 2 342 264**
**US - A - 3 965 192**

**CHEMICAL ABSTRACTS, Band 79, 1973, Seite 458, rechte Spalte, Nr. 92400n Columbus, Ohio, U.S.A. K. KOGAMI et al.: "Hydroformylation of limonene"**

(73) Patentinhaber: **Henkel Kommanditgesellschaft auf Aktien**
**Postfach 1100 Henkelstrasse 67**
**D-4000 Düsseldorf 1 (DE)**

(72) Erfinder: **Hagen, Jens, Dr.**
**Dieselstrasse 5**
**D-6834 Ketsch (DE)**
Erfinder: **Bruns, Klaus, Dr.**
**Notburgaweg 6**
**D-4150 Krefeld-Traar (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 272 B1

# Verfahren zur Herstellung von Aldehyden und Verwendung der Verfahrensprodukte als Riechstoffe

Die Erfindung betrifft ein Verfahren zur Herstellung von Aldehyden, die sich von monocyclischen und bicyclischen Monoterpenkohlenwasserstoffen ableiten, sowie die Verwendung der Verfahrensprodukte als Riechstoffe.

Bei der Hydroformylierung von Terpenen unter den bei der Oxo-Reaktion üblichen Bedingungen und in Gegenwart von Dicobaltoctacarbonyl entstehen komplexe Produktgemische, die Aldehyde, Alkohole, Acetale, Ether und Umlagerungsprodukte der jeweiligen Terpenkohlenwasserstoffe enthalten (W. H. Clement et al., Ind. Eng. Chem. Prod. Res. Dev. Vol. 4 (1965) S. 283—286). Weniger komplizierte Verbindungsgemische wurden bei der Hydroformylierung von $\alpha$-Pinen in Gegenwart von Rhodiumkatalysatoren erhalten (W. Himmele et al., Tetrahydron Letters *1976*, S. 907—910). Das Reaktionsgemisch enthielt in diesem Fall neben dem gewünschten 3-Formylpinan einen erheblichen Anteil an den beiden isomeren 10-Formylpinanen, die nur dann entstehen können, wenn unter den Reaktionsbedingungen eine Isomerisierung der Doppelbindung des $\alpha$-Pinens eintritt.

Aus der DE—A—1 939 322 ist ein Verfahren zur Hydroformylierung von olefinisch ungesättigten Verbindungen unter Verwendung von Hydridocarbonyl - bis - (trisubstituiertes Phosphin) - rhodium oder Hydridocarbonyl - tris - (trisubstituiertes Phosphin) - rhodium als Katalysator bekannt. Im Zusammenhang mit der Hydroformylierung von Pent - 1 - en wird dort die Verwendung eines Überschusses an trisubstituiertem Phosphin erwähnt. Die im experimentellen Teil ebenfalls beschriebene Hydroformylierung des Terpens dl-Limonen wurde jedoch nur in Gegenwart von $RH(CO)(PPh_3)_3$ durchgeführt.

Aus der US—A—3 956 192 ist ein Verfahren zur Hydroformylierung von monocyclischen $C_4$—$C_{18}$ - Monoolefinen bekannt, bei dem als Katalysatoren Rhodiumcarbonylverbindungen eingesetzt werden, die drei substituierte Phosphinliganden enthalten. Dabei kann das substituierte Phosphin gegebenenfalls im Überschuss vorhanden sein. Die Hydroformylierung von Terpenkohlenwasserstoffen wird in dieser Druckschrift nicht erwähnt.

Die US—A—3 499 932 beschreibt ein Verfahren zur Hydroformylierung von polycyclischen, nicht-konjugierten Diolefinen, deren beide Doppelbindungen im cycloaliphatischen Kern liegen. Als Katalysatorsystem wird dabei ein Gemisch aus einem Rhodiumcarbonyl-Triphenylphosphinkomplex und überschüssigem Triphenylphosphin verwendet. Auch hier wird die Hydroformylierung von monocyclischen und bicyclischen Monterpenkohlenwasserstoffen nicht erwähnt.

Aus Chemical Abstract, Band 79, 1973, Seite 458, rechte Spalte, Nr. 92 400 n ist die Hydroformylierung von Limonen in Gegenwart von $[Co(CO)_3 PBu_3]_2$, $[Co(CO)_3 PPh_3]_2$ oder phosphinfreien Rhodiumcarbonylkomplexen bekannt.

In der veröffentlichten prioritätsälteren EP—A—0 008 459, in der die Vertragsstaaten Bundesrepublik Deutschland, Frankreich, Großbritannien, Niederlande und Schweden benannt sind, wird die Herstellung von 2 - (4 - Methyl - 3 - cyclohexen - 1 - yl) butyraldehyd durch Hydroformylierung von Limonen in Gegenwart eines Gemisches aus Rhodiumcarbonylkomplexen, die zusätzlich als Liganden Triphenyl- oder Trialkylphosphin enthalten, und überschüssigem Phosphin beschrieben und beansprucht.

Es wurde nun gefunden, daß die Isomerisierung der in den Terpenkohlenwasserstoffen vorhandenen Doppelbindungen unter den Bedingungen der Hydroformylierungsreaktion weitgehend unterdrückt werden kann, wenn man ein aus tertiären organischen Phosphinen und Rhodiumcarbonylverbindungen bestehendes Katalysatorgemisch einsetzt.

Gegenstand der Erfindung ist demnach ein Verfahren zur Herstellung von Aldehyden durch Hydroformylierung von monocyclischen und bicyclischen Monoterpenkohlenwasserstoffen mit mindestens einer Doppelbindung bei 70 bis 160°C unter einem Druck von 100 bis 400 bar und in Gegenwart von Rhodiumcarbonylkomplexen, die tertiäre Phosphine enthalten, das dadurch gekennzeichnet ist, daß man die Hydroformylierung in Gegenwart eines Katalysatorgemisches aus tertiären Phosphinen und diese tertiären Phosphine enthaltenden Rhodiumcarbonylkomplexen durchführt.

Als tertiäre Phosphine eignen sich Trialkylphosphine, deren Alkylreste 1 bis 20 Kohlenstoffatome aufweisen, sowie Triphenylphosphine, deren Phenylreste durch Alkyl- oder Alkoxygruppen mit 1 bis 4 Kohlenstoffatomen substituiert sein können, insbesondere jedoch Triphenylphosphin. In den Katalysatormischungen liegt die Molzahl von insgesamt vorhandenem Phosphin pro Grammatom Rhodium im Bereich von 20 bis 200.

Die genaue Zusammensetzung der katalytisch wirksamen Rhodiumcarbonylkomplexe ist nicht bekannt. Man kann jedoch davon ausgehen, daß es sich um Rhodiumkomplexe handelt, in denen ein oder mehrere Carbonylliganden durch Phosphinliganden ersetzt sind. Die tatsächlich wirksame Komplexverbindung wird in jedem Fall in situ unter den Bedingungen der Hydroformylierung gebildet. Die hierzu erforderliche Menge Rhodium kann deshalb dem Reaktionsgemisch in Form von Rhodiumchlorid, Rhodiumoxid, Rhodiumsalzen von Fettsäuren, Rhodiumchelaten, Rhodiumcarbonyl oder dimeren Rhodiumcarbonylchlorid zugeführt werden. Vorzugsweise werden Rhodiumkomplexe eingesetzt, die das im Katalysatorgemisch vorhandene Phosphin bereits als Ligand enthalten, beispielsweise die Verbindung

$$RhCl(CO[P(C_6H_5)_3]_2.$$

Die Rhodiumverbindungen setzt man vorteilhaft in solchen Mengen ein, daß, bezogen auf den Terpenkohlenwasserstoff, 5 bis 5000 ppm, vorzugsweise 15 bis 400 ppm, berechnet als Metall, vorhanden sind.

Als Ausgangsmaterial für die Herstellung von Aldehyden nach dem erfindungsgemäßen Verfahren dienen monocyclische und bicyclische Monoterpenkohlenwasserstoffe mit mindestens einer Doppelbindung, wobei sowohl Verbindungen mit endocyclischen Doppelbindungen als auch solche mit semicyclischen und exocyclischen Doppelbindungen in Betracht kommen. Als Beispiele für solche Terpenkohlenwasserstoffe seien hier die isomeren Menthene, $\beta$-Terpinen, $\gamma$-Terpinen, $\alpha$-Phellandren, $\beta$-Phellandren, Pseudolimonen, Terpinolen und $\alpha$-Pinen sowie insbesondere Limonen, $\alpha$-Terpinen, $\beta$-Pinen und Camphen genannt. Auch natürlich vorkommende Gemische, die hauptsächlich solche Terpenkohlenwasserstoffe enthalten, beispielsweise Orangenöl, kommen als Ausgangsmaterial in Frage.

Die Umsetzung kann in Abwesenheit von Lösungsmitteln durch-geführt werden. Es hat sich jedoch als zweckmäßig erwiesen, Lösungsmittel zu verwenden, wobei u.a. gesättigte Kohlenwasserstoffe wie Pentan, Hexan, Heptan, Cyclohexan, Benzol, Toluol oder Xylol, Ether wie Tetrahydrofuran und Dioxan, Alkohole wie Methanol, Ethanol und Isopropanol, oder Diole wie Ethylenglykol und Propylenglykol in Frage kommen. Bevorzugt wird die Hydroformylierung in gesättigten Kohlenwasserstoffen oder Ethern durchgeführt.

Die Aufarbeitung des Reaktionsgemisches erfolgt auf dem Wege der Destillation, die zweckmäßigerweise in einer Inertgasatmosphäre, z.B. in einer Stickstoffatmosphäre, durchgeführt wird.

Die bei der beschriebenen Hydroformylierung entstehenden Reaktionsprodukte stellen in der Regel Gemische der möglichen Stereoisomeren der gewünschten Aldehydverbindung dar. Diese Gemische haben Riechstoffeigenschaften und können mit anderen Riechstoffen in verschiedenen Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition, bewegen. Derartige Kompositionen können direkt als Parfüm oder auch zur Parfümierung von Kosmetika, wie Cremes Lotionen, Duftwässern, Aerosolen, Toiletteseifen usw. dienen. Sie können aber auch zur Geruchsverbesserung von technischen produkten wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent zugesetzt.

Die nachfolgenden Beispiele sollen den Gegenstand der Erfindung näher erläutern, ohne ihn hierauf zu beschränken.

Beispiele

Beispiel 1

In einem 5 l - Hubrührautoklaven aus rostfreiem Stahl wurden 1360 g (10 mol) Limonen, 15,2 g (58 mmol) Triphenylphosphin und 0,4 g (0.58 mmol)

$$RhCl(CO)[P(C_6H_5)_3]_2$$

miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 125°C aufgeheizt, anschließend unter weiterem Rühren 3 Stunden lang auf dieser Temperatur gehalten, wobei der Druck auf maximal 270 bar anstieg, dann auf Raumtemperatur abgekühlt. Das erhaltene Rohprodukt (1660 g) wurde im Vakuum unter Stickstoffatmosphäre destilliert. Nach Abtrennen des nicht umgesetzten Limonens gingen bei 111—112°C/16 mbar 1381 g 3 - (4 - Methyl - 3 - cyclohexenyl) butyraldehyd (83% d.Th.) über.

Die Jodzahl des Produktes betrug 155 (theor. 153). Nach der gaschromatographischen Untersuchung wies das Produkt eine Reinheit von 98% auf. Das Produkt zeigte folgendes IR-Spektrum (Film):

3005 $cm^{-1}$; 1680 $cm^{-1}$ ( 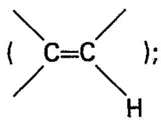 );

2710 $cm^{-1}$; 1726 $cm^{-1}$ (CHO).

Geruch: Agrumen-Note, Rhabarber-Note.

Beispiel 2

408 g (3 mol) Camphen, 7,6 g (29 mmol) Triphenylphosphin und 0,2 g (0,29 mmol)

$$RhCl(CO[P(C_6H_5)_3]_2$$

wurden in einem 5 1 - Hubrührautoklaven miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 120°C aufgeheizt und unter weiterem Rühren 4 Stunden lang auf 120 bis 130°C gehalten. Nach dem Abkühlen wurde das erhaltene Rohprodukt destillativ aufgearbeitet. Bei 105—106°C/20

mbar gingen 383 g 3,3 - Dimethyl - 2 - norbornanacetaldehyd (77% d.Th.) über.

Nach der gaschromatographischen Untersuchung bestand das Produkt zu 98% aus einem Gemisch des endo- und des exo-Stereoisomeren.

Das Produkt zeigte folgendes IR-Spektrum (Film):

2710 $cm^{-1}$; 1728 $cm^{-1}$ (CHO); 1385 $cm^{-1}$; 1365 $cm^{-1}$ (gem. di-Methyl).

Geruch: Borneol-Note; Campher-Note.

Beispiel 3

Ein 5 l - Hubrührautoklav wurde mit 272 g (2 mol) $\beta$ - Pinen, 7,6 g (29 mmol) Triphenylphosphin, 0,2 g (0,29 mmol)

$$RhCl(CO)[P(C_6H_5)_3]_2$$

und 750 ml Tetrahydrofuran beschickt und mit Synthesegas gespült. Anschließend wurde ein aus gleichen Volumina Wasserstoff und Kohlenmonoxid bestehendes Gasgemisch bis zu einem Druck von 200 bar aufgepreßt. Die Reaktionsmischung wurde unter Rühren auf 130°C aufgeheizt und 2 Stunden lang auf dieser Temperatur gehalten. Nach dem Abkühlen wurde des erhaltene Rohprodukt destillativ aufgearbeitet. Bei 105—107°C/10 mbar gingen 222 g 10-Formylpinan (67% d.Th.) über.

Nach der gaschromatographischen Untersuchung bestand das Produkt zu 97% aus einem Gemisch aus dem axialen und dem äquatorialen Stereoisomeren.

Das Produkt zeigte folgendes IR-Spektrum (Film):

2710 $cm^{-1}$; 1725 $cm^{-1}$ (CHO); 1368 $cm^{-1}$; 1381 $cm^{-1}$ (gem. di-Methyl).

Geruch: grün Citronellal-Note, Magriffe-Note.

Beispiel 4

In einem 5 l Hubrührautoklaven wurden 272 g (2 mol) $\alpha$ - Terpinen, 3,8 g (14,5 mmol) $P(C_6H_5)_3$, 0,2 g (0,29 mmol)

$$RhCl(CO)[P(C_6H_5)_3]_2$$

und 750 ml Tetrahydrofuran miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 130°C aufgeheizt, anschließend 5 Stunden lang auf 130—140°C gehalten, dann auf Raumtemperatur abgekühlt. Aus dem Reaktionsgemisch wurde Tetrahydrofuran im Wasserstrahlvakuum abdestilliert. Bei der Destillation des Rückstandes im Ölpumpenvakuum gingen bei 98—100°C/20 mbar 225 g Produkt (68% d.Th.) über.

Die gaschromatische Untersuchung zeigte, daß das Produkt ein Gemisch aus 2 - Formyl - Δ3 - menthen und 3 - Formyl - Δ1 - menthen darstellt.

Das Produkte zeigte folgendes IR-Spektrum (Film):

3005 $cm^{-1}$; 1682 $cm^{-1}$ ( 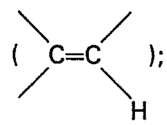 );

2700 $cm^{-1}$; 1725 $cm^{-1}$ (CHO); 1380 $cm^{-1}$; 1360 $cm^{-1}$ (Isopropyl); 845 $cm^{-1}$ (C=C trisubstituiert).

Geruch: Salicylat-Note, Cumin-Perilla-Note.

**Patentansprüche (DE, FR, GB, SE)**

1. Verfahren zur Herstellung von Aldehyden durch Hydroformylierung von monocyclischen und bicyclischen Monoterpenkohlenwasserstoffen mit mindestens einer Doppelbindungausgenommen Limonen bei 70 bis 160°C unter einem Druck von 100 bis 400 bar und in Gegenwart von Rhodiumcarbonylkomplexen, die tertiäre Phosphine enthalten, dadurch gekennzeichnet, daß man die Hydroformylierung in Gegenwart eines Katalysatorgemisches aus tertiären Phosphinen und diese tertiäre Phosphine enthaltenden Rhodiumcarbonylkomplexen.

2. Verfahren nach Anspruch 1 dadurch gekennzeichnet, daß das Katalysatorgemisch Trialkylphosphine, deren Alkylreste 1 bis 20 Kohlenstoffatome aufweisen, enthält.

3. Verfahren nach Anspruch 1, dadurch gekennzeichnet, daß das Katalysatorgemisch Triphenylphosphin oder Triphenylphosphine, deren Phenylreste durch Alkyl- oder Alkoxy-gruppen mit 1 bis 4 Kohlenstoffatomen substituiert sind, enthält.

4. Verfahren nach den Ansprüchen 1 bis 3, dadurch gekennzeichnet, daß in dem Katalysatorgemisch die Molzahl des insgesamt vorhandenen Phosphins pro Grammatom Rhodium im Bereich von 20 bis 200 liegt.

5. Verfahren nach den Ansprüchen 1, 3 und 4, dadurch gekennzeichnet, daß das Katalysatorgemisch aus Triphenylphosphin und

$$(Rh(CO)[P(C_6H_5)_3]_2$$

gebildet wird.

6. Verfahren nach den Ansprüchen 1 bis 5, dadurch gekennzeichnet, daß als Ausgangsmaterial für die Hydroformylierung Camphen, $\beta$-Pinen oder $\alpha$-Terpinen eingesetzt werden.

7. Verwendung der nach dem Verfahren der Ansprüche 1 bis 6 hergestellten Aldehyde als Riechstoffe.

8. Gemische wie sie bei der Hydroformylierung von Camphen $\beta$-Pinen oder $\alpha$-Terpinen bei 70 bis 160°C unter einem Druck von 100 bis 400 bar in Gegenwart eines Katalysatorgemisches aus tertiären Phosphinen und diese tertiären Phosphine enthaltenden Rhodium carbonylkomplexen erhalten werden.

**Patentansprüche (BE, CH, IT)**

1. Verfahren zur Herstellung von Aldehyden durch Hydroformylierung von monocyclischen und bicyclischen Monoterpenkohlenwasserstoffen mit mindestens einer Doppelbindung bei 70 bis 160°C unter einem Druck von 100 bis 400 bar und in Gegenwart von Rhodiumcarbonylkomplexen, die tertiäre Phosphine enthalten, dadurch gekennzeichnet, daß man die Hydroformylierung in Gegenwart eines Katalysatorgemisches aus tertiären Phosphinen und diese tertiäre Phosphine enthaltenden Rhodiumcarbonylkomplexen durchführt.

2. Verfahren nach Anspruch 1, dadurch gekennzeichnet, daß das Katalysatorgemisch Trialkylphosphine, deren Alkylreste 1 bis 20 Kohlenstoffatome aufweisen, enthält.

3. Verfahren nach Anspruch 1, dadurch gekennzeichnet, daß das Katalysatorgemisch Triphenylphosphin oder Triphenylphosphine, deren Phenylreste durch Alkyl- oder Alkoxygruppen mit 1 bis 4 Kohlenstoffatomen substituiert sind, enthalt.

4. Verfahren nach den Ansprüchen 1 bis 3, dadurch gekennzeichnet, daß in dem Katalysatorgemisch die Molzahl des insgesamt vorhandenen Phosphins pro Grammatom Rhodium im Bereich von 20 bis 200 liegt.

5. Verfahren nach den Ansprüchen 1, 3 und 4, dadurch gekennzeichnet, daß das Katalysatorgemisch aus Triphenylphosphin und

$$(Rh(CO)[P(C_6H_5)_3]_2$$

gebildet wird.

6. Verfahren nach den Ansprüchen 1 bis 5, dadurch gekennzeichnet, daß als Ausgangsmaterial für die Hydroformylierung Limonen, Camphen, $\beta$-Pinen oder $\alpha$-Terpinen eingesetzt werden.

7. Verwendung der nach dem Verfahren der Ansprüche 1 bis 6 hergestellten Aldehyde als Riechstoffe.

8. Gemische wie sie bei der Hydroformylierung von Limonen, Camphen, $\beta$-Pinen oder $\alpha$-Terpinen bei 70 bis 160°C unter einem Druck von 100 bis 400 bar in Gegenwart eines Katalysatorgemisches aus tertiären Phosphinen und diese tertiären Phosphine enthaltenden Rhodiumcarbonylkomplexen erhalten werden.

**Revendications (DE, FR, GB, SE) pour les Etats contractants**

1. Procédé de préparation d'aldéhydes par hydroformylation d'hydrocarbures monoterpéniques monocycliques et bicycliques contenant au moins une double liaison—à l'exception du limonène—à une température de 70 à 160°C, sous une pression de 100 à 400 bars et en présence de complexes à base de rhodium-carbonyle contenant des phosphines tertiaires, caractérisé en ce que l'on effectue l'hydroformylation en présence d'un mélange catalyseur consistant en phosphines tertiaires et complexes de rhodium-carbonyle contenant ces phosphines tertiaires.

2. Procédé selon la revendication 1, caractérisé en ce que le mélange catalyseur contient des trialkylphosphines dont les groupes alkyle sont en $C_1$—$C_{20}$.

3. Procédé selon la revendication 1, caractérisé en ce que le mélange catalyseur contient de la triphénylphosphine ou des triphénylphosphines dont les groupes phényle sont substitués par des groupes alkyle ou alcoxy en $C_1$—$C_4$.

4. Procédé selon les revendications 1 à 3, caractérisé en ce que, dans le mélange catalyseur, le nombre de moles de phosphine présente au total par atome-gramme de rhodium se situe dans l'intervalle de 20 à 200.

5. Procédé selon les revendications 1, 3 et 4, caractérisé en ce que le mélange catalyseur est constitué de triphenylphosphine et de

$$(Rh(CO)[P(C_6H_5)_3]_2.$$

6. Pocédé selon les revendications 1 à 5, caractérisé en ce que l'on utilise en tant que produits de départ de l'hydroformylation du camphène, du $\beta$-pinène ou de l'$\alpha$-terpinène.

7. Utilisation des aldéhydes préparés par le procédé des revendications 1 à 6 en tant que matières aromatiques.

8. Mélanges tels qu'obtenus à l'hydroformylation du camphène, du $\beta$-pinène ou de l'$\alpha$-terpinène à des température de 70 à 160°C, sous une pression de 100 à 400 bars en présence d'un mélange catalyseur consistant en phosphines tertiaires et complexes de rhodium-carbonyle contenant ces phosphines tertiaires.

**Revendications (BE, CH, IT) pour les Etats contractants:**

1. Procédé de préparation d'aldéhydes par hydroformylation d'hydrocarbures monoterpéniques monocycliques et bicycliques contenant qu moins une double liaison à une température de 70 à 160°C, sous une pression de 100 à 400 bars et en présence de complexes à base de rhodium-carbonyle contenant des phosphines tertiaires, caractérisé en ce que l'on effectue l'hydroformylation en présence d'un mélange catalyseur consistant en phosphines tertiaires et complexes de rhodiumcarbonyle contenant ces phosphines tertiaires.

2. Procédé selon la revendication 1, caractérisé en ce que le mélange catalyseur contient des trialkylphosphines dont les groupes alkyle sont en $C_1$—$C_{20}$.

3. Procédé selon la revendication 1, caractérisé en ce que le mélange catalyseur contient de la triphénylphosphine ou des triphénylphosphines dont les groupes phényle sont substitués par des groupes alkyle ou alcoxy en $C_1$—$C_4$.

4. Procédé selon les revendications 1 à 3,

caractérisé en ce que, dans le mélange catalyseur, le nombre de moles de phosphine présente au total par atome-gramme de rhodium se situe dans l'intervalle de 20 à 200.

5. Procédé selon les revendications 1, 3 et 4, caractérisé en ce que le mélange catalyseur est constitué de triphénylphosphine et de

$$(Rh(CO)[P(C_6H_5)_3]_2.$$

6. Procédé selon les revendications 1 à 5, caractérisé en ce que l'on utilise en tant que produits de départ de l'hydroformylation du limonène, du camphène, du $\beta$-pinène ou de l'$\alpha$-terpinène.

7. Utilisation des aldéhydes préparés par le procédé des revendications 1 à 6 en tant que matières aromatiques.

8. Mélanges tels qu'obtenus à l'hydroformylation du limonène, du camphène, du $\beta$-pinène ou de l'$\alpha$-terpinène à des températures de 70 à 160°C, sous une pression de 100 à 400 bars en présence d'un mélange catalyseur consistant en phosphines tertiaires et complexes de rhodium-carbonyle contenant ces phosphines tertiaires.

**Claims for the Contracting States: (DE, FR, GB, SE)**

1. A process for the preparation of aldehydes by the hydroformylation of monocyclic and bicyclic monoterpene hydrocarbons—with the exception of limonene—containing at least one double bond at 70 to 160°C under a pressure of from 100 to 400 bars and in the presence of rhodium carbonyl complexes containing tertiary phosphines, characterised in that the hydroformylation reaction is carried out in the presence of a catalyst mixture of tertiary phosphines and rhodium carbonyl complexes containing these tertiary phosphines.

2. A process as claimed in Claim 1, characterised in that the catalyst mixture contains trialkyl phosphines of which the alkyl radicals contain from 1 to 20 C-atoms.

3. A process as claimed in Claim 1, characterised in that the catalyst mixture contains triphenyl phosphine or triphenyl phosphines of which the phenyl radicals are substituted by alkyl or alkoxy groups containing from 1 to 4 C-atoms.

4. A process as claimed in Claims 1 to 3, characterised in that, in the catalyst mixture, the number of mols of the total phosphine present per gram-atom of rhodium is in the range from 20 to 200.

5. A process as claimed in Claims 1, 3 and 4, characterised in that the catalyst mixture consists of triphenyl phosphine and

$$(Rh(CO)[P(C_6H_5)_3]_2.$$

6. A process as claimed in Claims 1 to 5, characterised in that camphene, $\beta$-pinene or $\alpha$-terpinene are used as starting material for the hydroformylation reaction.

7. The use of aldehydes obtained by the process claimed in Claims 1 to 6 as fragrances.

8. Mixtures obtained in the hydroformylation of camphene, $\beta$-pinene or $\alpha$-terpinene at 70 to 160°C under a pressure of from 100 to 400 bars in the presence of a catalyst mixture of tertiary phosphines and rhodium carbonyl complexes containing these tertiary phosphines.

**Claims for the Contracting States: (BE, CH, IT)**

1. A process for the preparation of aldehydes by the hydroformylation of monocyclic and bicyclic monoterpene hydrocarbons containing at least one double bond at 70 to 160°C under a pressure of from 100 to 400 bars and in the presence of rhodium carbonyl complexes containing tertiary phosphines, characterised in that the hydroformylation reaction is carried out in the presence of a catalyst mixture of tertiary phosphines and rhodium carbonyl complexes containing these tertiary phosphines.

2. A process as claimed in Claim 1, characterised in that the catalyst mixture contains trialkyl phosphines of which the alkyl radicals contain from 1 to 20 C-atoms.

3. A process as claimed in Claim 1, characterised in that the catalyst mixture contains triphenyl phosphine or triphenyl phosphines of which the phenyl radicals are substituted by alkyl or alkoxy groups containing from 1 to 4 C-atoms.

4. A process as claimed in Claims 1 to 3, characterised in that, in the catalyst mixture, the number of mols of the total phosphine present per gram-atom of rhodium is in the range from 20 to 200.

5. A process as claimed in Claims 1, 3 and 4, characterised in that the catalyst mixture consists of triphenyl phosphine and

$$(Rh(CO)[P(C_6H_5)_3]_2.$$

6. A process as claimed in Claims 1 to 5, characterised in that limonene, camphene, $\beta$-pinene or $\alpha$-terpinene are used as starting material for the hydroformylation reaction.

7. The use of the aldehydes obtained by the process claimed in Claims 1 to 6 as fragrances.

8. Mixtures obtained in the hydroformylation of limonene, camphene, $\beta$-pinene or $\alpha$-terpinene at 70 to 160°C under a pressure of from 100 to 400 bars in the presence of a catalyst mixture of tertiary phosphines and rhodium carbonyl complexes containing these tertiary phosphines.